# EUROPÄISCHE PATENTSCHRIFT

(11) **EP 0 920 287 B1**
(45) Veröffentlichungstag und Bekanntmachung des Hinweises auf die Patenterteilung: **21.07.2004**
(21) Anmeldenummer: 98936117.5
(22) Anmeldetag: 09.06.1998
(51) Int. Cl.: A61F 2/14, A61F 2/16

(54) **BLENDE ZUM IMPLANTIEREN IN DEN LINSENKAPSELSACK EINES AUGES**
DIAPHRAGM WHICH CAN BE IMPLANTED IN THE CRYSTALLINE LENS CAPSULE OF THE EYE
DIAPHRAGME A IMPLANTER DANS LA CAPSULE DU CRISTALLIN D'UN OEIL

(30) Priorität: 11.06.1997 DE 19724539
(43) Veröffentlichungstag der Anmeldung: 09.06.1999
(73) Patentinhaber: Hermeking, Heino, 42369 Wuppertal (DE)
(72) Erfinder: Hermeking, Heino, 42369 Wuppertal (DE)
(74) Vertreter: Meyer-Roedern, Giso, Dr.
(86) Internationale Anmeldenummer: PCT/DE1998/001566
(87) Internationale Veröffentlichungsnummer: WO 1998/056314

(56) Entgegenhaltungen:
- EP-A- 0 319 154
- WO-A-95/28897
- DE-A- 3 926 536
- FR-A- 2 696 340
- US-A- 5 628 797

## Beschreibung

Die Erfindung betrifft eine Einfachblende, eine Doppelblende und einen Bausatz derartiger Blenden, die sich zur Schaffung einer künstlichen Pupillenöffnung in den Linsenkapselsack eines Auges implantieren lassen.

Es ist Stand der medizinischen Technik, den grauen Star (Katarakt) durch operatives Entfernen der getrübten Linse zu behandeln und eine künstliche Intraokularlinse in den Linsenkapselsack des Auges einzusetzen.

Zur Versorgung von Irisdefizienzen sind Aniridie-Intraokularlinsen bekannt, die nur im Bereich einer zentralen künstlichen Pupillenöffnung transparent im Sinne einer dioptrischen Optik und an der Peripherie eingefärbt sind.

Die Verwendung einer Aniridie-Intraokularlinse kommt bei Vorliegen kleinerer Defekte der Iris nicht zwingend in Betracht. Ein Nachteil dieser fixen Kombination von Blende mit dioptrischer Optik ist die im allgemeinen schlechte oder nicht unmittelbare Verfügbarkeit dieser Prothetik. Dies ist zum einen in der komplizierten Herstellung begründet und zum anderen in lagerungstechnischen Gründen, da es zu aufwendig und ökonomisch untragbar wäre, sämtliche dioptrischen Stärken für Fälle, die nicht der Routine entsprechen, auf Vorrat zu halten.

Es ist bekannt, insbesondere bei Komplikationen als weitere Prothese zusätzlich zu der Intraokularlinse einen Spannring in den Linsenkapselsack einzuführen, um ihn auszuspannen und zu stabilisieren und den Halteapparat der Linsenkapsel zu entlasten. Dies geschieht durch Druck des Kapselspannringes auf den Äquatorbereich des Kapselsackes.

Bei Vorliegen einer Aniridie oder eines Iriskolobomes besteht auch das prothetische Angebot eines Kapselspannringes mit einer oder mehreren nach innen vorstehenden Blende(n) zur Implantation in den Kapselsack, die eine zentrale künstliche Pupillenöffnung freilassen sollen.

Die Kombination von Kapselspannring und Blende geht in ihrer Zielsetzung, nämlich eine artifizielle Pupille bestimmter Größe zu schaffen, daran vorbei, daß ihr "Wirkprinzip" entgegengesetzt ist. Während der Spannring seine Spannung auf den Äquatorbereich des Kapselsackes ausübt, liegt die Zielrichtung der an ihm befestigten Blenden in Richtung Kapselsackmitte. Dies ist der Fall, um eine artifizielle Pupillenapertur definierter Große zu erhalten. Aufgrund der Spannung können sich die Blenden hinter die Skleragrenzen (Limbus) verschieben, und es kann sich eine zu große künstliche Pupillenöffnung ergeben. Das Ergebnis dieser Implantate ist, was die Pupillenapertur angeht, stark abhängig von den anatomischen Kapselsackgegebenheiten und nicht vorhersagbar. Damit erfüllt diese Prothetik die Zielsetzung, eine Pupillenöffnung definierter Größe zu schaffen, nicht.

Aus der US-A-5 628 797 ist eine Blende bekannt, die in die Vorderkammer eines Auges implantiert werden kann. Die Blende ist aus zwei gelenkig miteinander verbundenen, halbkreisförmigen Segmenten zusammengesetzt.

Die EP-A-0 319 154 beschreibt den zweistückigen Aufbau einer Intraokularlinse mit einem Spannrahmen und einem davon gehalterten Linsenkörper.

Aufgabe der Erfindung ist es, eine Einfachblende, Doppelblende und einen Bausatz derartiger Blenden zum Implantieren in den Linsenkapselsacks eines Auges zu schaffen, die von einem Kapselspannring funktionell entkoppelt sind und eine Pupillenöffnung definierter Größe schaffen.

Gelöst wird diese Aufgabe zum einen mit einer Einfachblende, die sich zusätzlich zu einer künstlichen Linse im wesentlichen spannungsfrei in den Linsenkapselsack eines Auges implantieren läßt. Die Einfachblende hat einen Überdeckungsbereich einseitig neben einem zentralen Freiraum und eine bügelförmige Haptik auf der anderen Seite davon.

Die Haptik ist vorzugsweise C-förmig oder J-förmig.

Gelöst wird die vorgenannte Aufgabe zum anderen mit einer Doppelblende, die sich zusätzlich zu einer künstlichen Linse im wesentlichen spannungsfrei in den Linsenkapselsack eines Auges implantieren läßt. Die Doppelblende hat zwei einander gegenüberliegende Überdeckungsbereiche beidseits eines zentralen Freiraums und einen die Überdeckungsbereiche verbindenden elastischen Bügel seitlich daneben.

Der Bügel ist vorzugsweise U-förmig nach innen gewölbt.

Die erfindungsgemäßen Blenden sind vorzugsweise insgesamt flach. Sie können aber auch einen oder mehrere flache Überdeckungsbereiche und eine davon abgewinkelte Haptik haben.

Bei einer bevorzugten Ausführungsform läßt sich in die Blenden ein Stikker einsetzen, der die Blenden nach erfolgter Implantation in den Linsenkapselsack aussteift.

Die Blenden können mit einer oder mehreren Öffnungen für eine Nadelspitze oder einen Führungshaken versehen sein.

Die Blenden bestehen vorzugsweise aus gefärbtem PMMA (Polymethylmethacrylat) oder Polycarbonat. Sie können aber auch aus einem faltbaren gefärbten Material, insbesondere Silikon oder Weichacryl, bestehen.

Die Konzeption dieser Blenden beruht darauf, daß der Kapselsackäquator primär keine Kraft auf die (kompressiblen) Blenden ausübt. Dies hat den Vorteil, daß die durch das Design vorgegebenen Pupillengrößen erhalten bleiben. Weitere Vorteile der vorgelegten Konzeptionen ist die unkomplizierte Implantierbarkeit und die unmittelbare Verfügbarkeit durch die Unabhängigkeit von einer Optik.

Gelöst wird die vorgenannte Aufgabe zum dritten durch einen Bausatz Einfachblenden, Doppelblenden oder einer oder mehrerer Einfach- und Doppelblende (n) der vorgenannten Art. Die Einfach- und/oder Doppelblende(n) lassen sich winkelversetzt übereinanderliegend in den Kapselsack implantieren, wobei sie einander derart überdecken, daß eine zentrale, annähernd kreisrunde künstliche Pupillenöffnung freibleibt.

Die Erfindung sieht je einen Bausatz Blenden für eine künstliche Pupillenöffnung von 3 mm und 4 mm Durchmesser vor.

Bei einer bevorzugten Ausführungsform lassen sich die Blenden des Bausatzes intrakapsulär miteinander verbinden, insbesondere miteinander verspannen, verklipsen oder verrasten.

Bei einer bevorzugten Ausführungsform ist ein Fixierring mit einer kreisrunden Apertur zum Einklinken in eine oder mehrere Blende(n) der genannten Art vorgesehen. Durch den Fixierring wird eine exakt kreisrunde künstliche Pupillenöffnung geschaffen.

Die Erfindung wird im folgenden anhand von in der Zeichnung dargestellten Ausführungsbeispielen näher erläutert. Es zeigen:

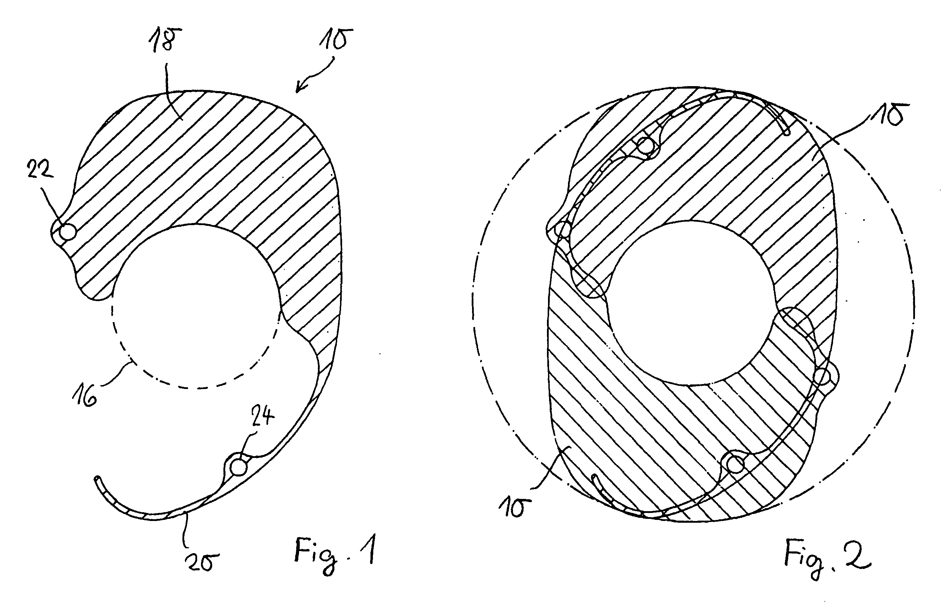

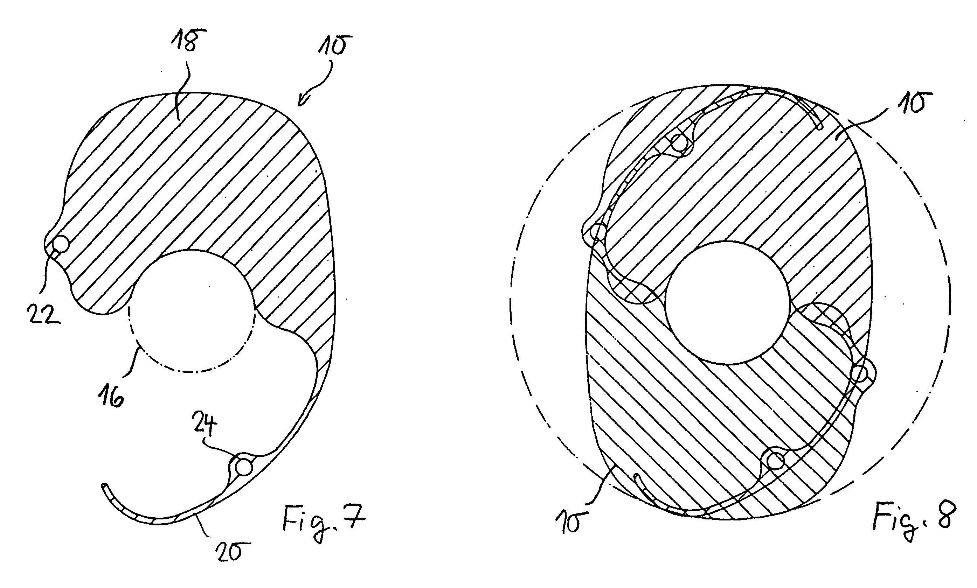

- Fig. 1: bis Fig. 6 einen ersten Bausatz Blenden zur Schaffung einer künstlichen Pupillenöffnung von 4 mm Durchmesser, und zwar
- Fig. 1: in Draufsicht eine Einfachblende;
- Fig. 2: in Draufsicht zwei Einfachblenden gemäß Fig. 1, die um 180° versetzt miteinander in Anlage stehen;
- Fig. 3: in Draufsicht eine Doppelblende;
- Fig. 4: in Draufsicht zwei Doppelblenden gemäß Fig. 3, die um 90° versetzt miteinander in Anlage stehen;
- Fig. 5: in Draufsicht eine abgewandelte Einfachblende;
- Fig. 6: in Draufsicht eine abgewandelte Doppelblende;
- Fig. 7: bis 12 einen zweiten Bausatz Blenden zur Schaffung einer künstlichen Pupillenöffnung von 3 mm Durchmesser, und zwar
- Fig. 7: in Draufsicht eine Einfachblende;
- Fig. 8: in Draufsicht zwei Einfachblenden gemäß Fig. 7, die um 180° versetzt miteinander in Anlage stehen;

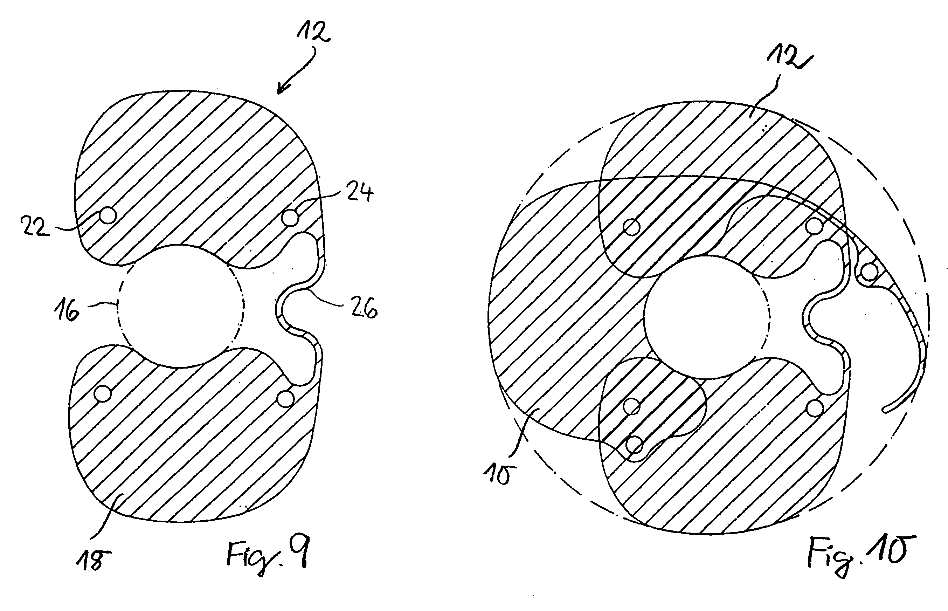

- Fig. 9: in Draufsicht eine Doppelblende;
- Fig. 10: in Draufsicht eine Einfachblende gemäß Fig. 7 und eine Doppelblende gemäß Fig. 9, die um 90° versetzt miteinander in Anlage stehen;

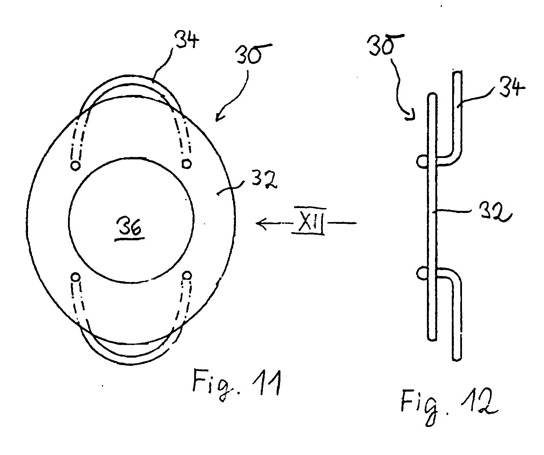

- Fig. 11: in Draufsicht einen Fixierring für die Blenden; und
- Fig. 12: eine Seitenansicht des Fixierrings mit Blick in Richtung XII von Fig. 11.

Die Blenden 10, 12 sind Flachteile aus gefärbtem PMMA.

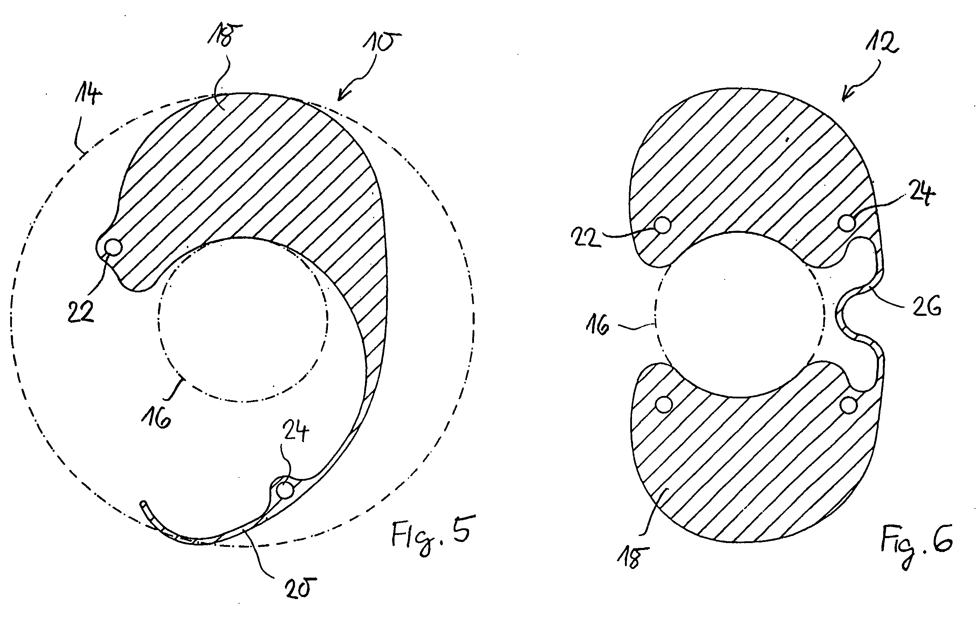

Die Blenden gemäß Fig. 1 bis Fig. 4 und Fig. 7 bis Fig. 10 passen spannungsfrei in einen Umkreis 14 von 10,5 mm Durchmesser. Die Blenden 10, 12 gemäß Fig. 5 und Fig. 6 passen spannungfrei in einen Umkreis 14 von 11 mm Durchmesser.

Mit dem Bausatz Blenden 10, 12 gemäß Fig. 1 bis Fig. 6 wird eine künstliche Pupillenöffnung 16 mit 4 mm Durchmesser geschaffen. Bei dem Bausatz Blenden gemäß Fig. 7 bis Fig. 10 beträgt der Durchmesser der künstlichen Pupillenöffnung 16 3 mm.

Die in Fig. 1 und Fig. 2 gezeigte Einfachblende 10 hat einen Überdeckungsbereich 18 einseitig neben der künstlichen Pupillenöffnung 16. Der Überdeckungsbereich 18 erstreckt sich von dem Rand der künstlichen Pupillenöffnung 16 bis an den Umkreis 14.

Die Einfachblende 10 hat eine C-förmige Haptik 20, die den Umkreis 14 dem Überdeckungsbereich 18 diametral gegenüber tangiert. Das Ende der Haptik 20 ist von dem Umkreis 14 weg nach innen gekrümmt.

Die Haptik 20 setzt einseitig an dem Überdeckungsbereich 18 an. Sie verschmalert sich zu ihrem Ende hin, und sie läßt die künstliche Pupillenöffnung 16 frei.

Am haptikfreien Rand des Überdeckungsbereichs 18 ist eine Öffnung 22 für einen Führungshaken vorgesehen. Eine weitere solche Öffnung 24 befindet sich an der Haptik 20 nahe ihrem Berührende mit dem Umkreis 14. Die Öffnungen 22, 24 liegen einander etwa gegenüber.

Bei Implantation zusammen mit einer künstlichen Linse in den Kapselsack dient die Einfachblende 10 für sich allein zur Abdeckung von Iriskolobomen (Irisspalten).

Wie in Fig. 2 gezeigt, wird bei doppelter Implantation der Blende 10 in den Kapselsack eine Pupillenbildung erreicht. Die beiden Einfachblenden 10 stehen um 180° versetzt miteinander in bündiger Anlage.

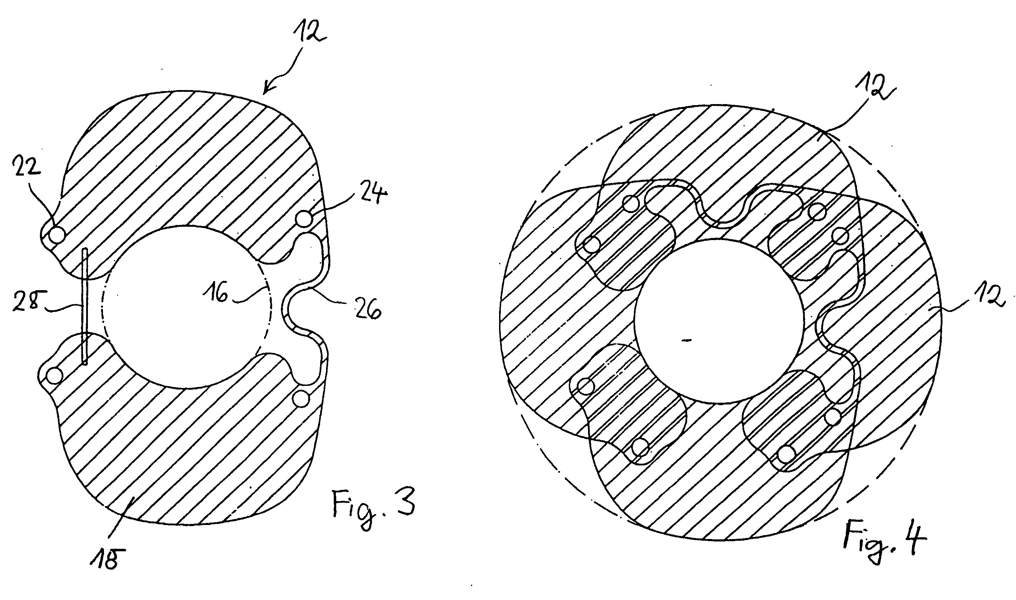

Die in Fig. 3 gezeigte Doppelblende 12 hat zwei formgleiche Überdeckungsbereiche 18, die einander diametral gegenüberliegen und sich jeweils vom Rand der künstlichen Pupillenöffnung 16 bis an den Umkreis 14 erstrecken. Die Überdeckungsbereiche 18 sind auf der einen Seite der künstlichen Pupillenöffnung 16 durch einen elastischen Bügel 26 verbunden, der U-förmig nach innen gewölbt ist und die künstliche Pupillenöffnung 16 freiläßt.

Die Doppelblende 12 läßt sich mit einem Sticker 28 aussteifen, der sich auf der dem Bügel 26 gegenüberliegenden Seite befindet und die Überdeckungsbereiche 18 verbindet. Der als gerader Stift ausgebildete Sticker 28 sitzt in einander gegenüberliegenden Sacklöchern der Überdeckungsbereiche 18, die sich in der Hauptebene der Blende 12 befinden und in deren entspanntem Zustand fluchten. Der Sticker 28 kann mit einem der Überdeckungsbereiche 18 von vornherein verbunden und insbesondere auch einstückig damit ausgebildet sein. Er wird nach dem Implantieren der Blende 12 in den Linsenkapselsack in den anderen Überdeckungsbereich 18 eingesteckt.

Die Überdeckungsbereiche 18 haben an ihrem haptikfreien Rand und am Bügelansatz je eine Öffnung 22, 24 für eine Nadelspitze.

Beim Implantieren zusammen mit einer künstlichen Linse in den Linsenkapselsack dient die Doppelblende 12 für sich allein zur Abdeckung von gegenüberliegenden Irisdefekten mit geringer Ausdehnung. Ein Beispiel sind iatrogene Sphinkterotomien, die zur Behandlung enger Pupillen vor Durchführung einer Kataraktoperation angelegt wurden.

Wie in Fig. 4 gezeigt, läßt sich mit zwei hintereinanderliegenden, um 90° zueinander versetzten Doppelblenden gemäß Fig. 3 eine annähernd kreisrunde künstliche Pupillenöffnung 16 schaffen.

Fig. 5 und Fig. 6 zeigen eine abgewandelte Einfachblende 10 und Doppelblende 12 mit etwas kleinerem Überdeckungsbereich 18 und etwas größerem Umkreis 14.

Bei den Blenden gemäß Fig. 7 bis Fig. 10 ist der Durchmesser der künstlichen Pupillenöffnung 16 etwas kleiner.

Wie in Fig. 10 gezeigt, sind auch die Einfach- und Doppelblende 10, 12 zur gemeinsamen Implantation in den Kapselsack miteinander kompatibel. Das gilt für alle Blenden 10, 12 mit gleicher Größe der künstlichen Pupillenöffnung 16.

Fig. 11 und Fig. 12 zeigen einen Fixierring 30 für die Blenden 10, 12. Der Fixierring 30 besteht aus gefärbtem PMMA. Er hat einen flachen ovalen Ringkörper 32, an dessen Breitseiten zwei Haptikbügel 34 senkrecht aus der Ringebene hoch und parallel dazu nach außen abgewinkelt sind. Der Fixierring 30 wird mit den Haptikbügeln 34 an den Blenden 10, 12 eingeklinkt. Er hat eine mittige kreisrunde Apertur 36 von 3 mm Durchmesser.

Die Erfindung stellt ein Konzept für kapsuläre Irisblenden ohne Spannung vor. Es wird ein Bausteinsystem für den artifiziellen Irisersatz geschaffen.

Das Design der Bausteine für den artifiziellen Irisaufbau ist in der Weise konzeptioniert, daB die Pupillengröße, die nach der Implantation der Bausteine in den Kapselsack vorliegt, in der Gestaltung der Bausteine vorgegeben ist.

Dies bedeutet bei der Annahme, daß der menschliche Kapselsack einen Außendurchmesser von 10,5 mm bis 11 mm besitzt, daß für den spannungsfreien Sitz der Bausteine ein Außendurchmesser dieser Prothetik von 11 mm nicht überschritten werden sollte.

Eine Ausnahme bildet hier der Fall von Myopie, bei dem für den Kapselsack Außendurchmesser von 12 mm und mehr erwartet werden können. Für solche Fälle excessiver Myopie können die Bausteine entsprechend größer sein.

Eine durch die Bausteinkonfiguration vorgegebene Pupillengröße kann bei flexiblen Bausteinen, wie sie im Design vorliegen, nur dann im Kapselsack erzielt werden, wenn vom Kapselsackäquator keine Spannungen auf die Prothesen ausgeübt werden. Das erfindungsgemäße Bausteinsystem erfüllt diese Bedingungen, so daß im frisch operierten Zustand die vorgegebene Pupillengröße erreicht wird.

Da Kapselsäcke postoperativ bekanntlich Schrumpfungstendenzen zeigen, ist nicht auszuschließen, daß eine sekundäre Spannung auf die Bausteine ausgeübt wird. Damit die Bausteine dem ohne Verformung standhalten, ist es denkbar, sie im Kapselsack auszusteifen und/oder miteinander zu verbinden, was beispielsweise durch Verspannen, Verklipsen oder Verrasten geschehen kann. Die Bausteine können insbesondere mit einem Sticker ausgesteift werden und/oder nach dem Stecker-Steckbuchsen-Prinzip miteinander in Eingriff gehen.

Das Aussteifen und/oder miteinander Verbinden der Bausteine kann aus implantationstechnischen Gründen nur im Kapselsack erfolgen.

Die Bausteine erreichen ihre Stabilität im Kapselsack einerseits durch ihr Design und andererseits durch das Implantieren schichtweise übereinander.

Mit der C-Haptik oder J-Haptik oder auch mit den beiden Überdeckungsbereichen 18 der Doppelblende 12 wird jeweils eine die Kapselsackgröße erreichende Ausdehnung der Bausteine vorgegeben. Dadurch, daß die Bausteine mit ihren Enden im Äquatorbereich des Kapselsackes liegen, ist bei gleichzeitiger Schichtung der Implantate die Stabilität ihrer Position im frischen operativen und postoperativen Zustand gewährleistet.

Als Material wird gegenwärtig PMMA mit eingelassenem Pigmentfarbstoff (Blau, Grün, Braun) oder Polykarbonat eingesetzt.

Es ist auch der Einsatz anderer biokompatibler Materialien denkbar, z. B. Silikon oder Weichacryl. Voraussetzung ist eine Einfärbung dieser Materialien.

Aufgrund der Tatsache, daß diese Materialien nicht nur kompressibel, sondern auch faltbar sind, kommt als Blende auch ein kreisrunder Ring mit einem Außendurchmesser von etwa 10,5 mm und einem Pupillendurchmesser von z. B. 3 mm oder 4 mm als kapsulärer artifizieller Iriseinsatz (Ersatz) in Betracht.

### Liste der Bezugszeichen

- 10: Einfachblende
- 12: Doppelblende
- 14: Umkreis
- 16: künstliche Pupillenöffnung
- 18: Überdeckungsbereich
- 20: Haptik
- 22: Öffnung
- 24: Öffnung
- 26: Bügel
- 28: Sticker
- 30: Fixierring
- 32: Ringkörper
- 34: Haptikbügel
- 36: Apertur

## Patentansprüche

1. Einfachblende (10) zur Schaffung einer künstlichen Pupillenöffnung, die zusätzlich zu einer künstlichen Linse im wesentlichen spannungsfrei in den Linsenkapselsack eines Auges implantierbar ist, mit einem Überdeckungsbereich (18) einseitig neben einem zentralen Freiraum (16) und einer bügelförmigen Haptik (20) auf der anderen Seite davon.

2. Einfachblende (10) nach Anspruch 1, **dadurch gekennzeichnet, daß** die Haptik (20) C-förmig oder J-förmig ist.

3. Doppelblende (12) zur Schaffung einer künstlichen Pupillenöffnung, die zusätzlich zu einer künstlichen Linse im wesentlichen spannungsfrei in den Linsenkapselsack eines Auges implantierbar ist, mit zwei einander gegenüberliegenden Überdeckungsbereichen (18) beidseits eines zentralen Freiraums (16) und mit einem die Überdeckungsbereiche (18) verbindenden elastischen Bügel (26) seitlich daneben.

4. Doppelblende (12) nach Anspruch 3, **dadurch gekennzeichnet, daß** der Bügel (26) U-förmig nach innen gewölbt ist.

5. Einfach- oder Doppelblende (10, 12) nach einem der Ansprüche 1 bis 4, **dadurch gekennzeichnet, daß** sie flach ist.

6. Einfach- oder Doppelblende (10, 12) nach einem der Ansprüche 1 bis 4, **dadurch gekennzeichnet, daß** sie wenigstens einen flachen Überdeckungsbereich (18) und eine davon abgewinkelte Haptik hat.

7. Einfach- oder Doppelblende (10, 12) nach einem der Ansprüche 1 bis 6, **dadurch gekennzeichnet, daß** ein die Einfach- oder Doppelblende (10, 12) aussteifender Sticker (28) darin einsetzbar ist.

8. Einfach- oder Doppelblende (10, 12) nach einem der Ansprüche 1 bis 7, **dadurch gekennzeichnet, daß** sie mit wenigstens einer Öffnung (22, 24) für eine Nadelspitze oder einen Führungshaken versehen ist.

9. Einfach- oder Doppelblende (10, 12) nach einem der Ansprüche 1 bis 8, **dadurch gekennzeichnet, daß** sie aus gefärbtem PMMA (Polymethylmethacrylat) oder Polycarbonat besteht.

10. Einfach- oder Doppelblende nach Anspruch 1 oder 3, **dadurch gekennzeichnet, daß** sie aus einem faltbaren gefärbten Material, insbesondere Silikon oder Weichacryl, besteht.

11. Bausatz Einfachblenden, Doppelblenden oder einer oder mehrerer Einfach- und/oder Doppelblende (n) (10, 12) nach einem der Ansprüche 1 bis 10, **dadurch gekennzeichnet, daß** die Einfach- und/oder Doppelblende(n) (10, 12) winkelversetzt übereinanderliegend in den Kapselsack implantierbar sind, wobei sie einander derart überdecken, daß eine zentrale, annahernd kreisrunde künstliche Pupillenöffnung (16) freibleibt.

12. Bausatz nach Anspruch 11 für eine künstliche Pupillenöffnung (16) von 3 mm Durchmesser.

13. Bausatz nach Anspruch 11 für eine künstliche Pupillenöffnung (16) von 4 mm Durchmesser.

14. Bausatz nach einem der Ansprüche 11 bis 13, **dadurch gekennzeichnet, daß** die Einfach- und/oder Doppelblende(n) (10, 12) intrakapsulär miteinander verbindbar, insbesondere miteinander verspannbar, verklipsbar oder verrastbar ist/sind.

15. Fixierring mit einem flachen ovalen Ringkörper (32), der eine mittige kreisrunde Apertur (36) hat und an dessen Breitseiten zwei Haptikbügel (34) senkrecht aus der Ringebene hoch und parallel dazu nach außen abgewinkelt sind, die an dem/den Überlappungsbereich(en) (18) einer oder mehrerer der Blende(n) (10, 12) nach einem der Ansprüche 1 bis 14 einklinkbar sind.

## Claims

1. Single diaphragm (10) for the creation of an artificial pupil aperture configured to be implanted in addition to an artificial lens substantially without stress in the lens capsule sac of an eye comprising an overlap region (18) on one side next to a central open space (16) and a stirrup-shaped haptic member (20) on the other side thereof.

2. Single diaphragm (10) according to claim 1, **characterized in that** the haptic member (20) is C-shaped or J-shaped.

3. Double diaphragm (12) for the creation of an artificial pupil aperture configured to be implanted in addition to an artificial lens substantially without stress in the lens capsule sac of an eye comprising two overlap regions (18) disposed opposite each other on both sides of a central open space (16) and an elastic stirrup (26) therebeside, joining the overlap regions (18).

4. Double diaphragm (12) according to claim 3, **characterized in that** the stirrup (26) is curved inwardly in the form of a U.

5. Single diaphragm (10) or double diaphragm (12) according to one of the claims 1 to 4, **characterized in that** it is flat.

6. Single diaphragm (10) or double diaphragm (12) according to one of the claims 1 to 4, **characterized in that** it has at least one flat overlap region (18) and a haptic member angled away therefrom.

7. Single diaphragm (10) or double diaphragm (12) according to one of the claims 1 to 6, **characterized in that** a stitch (28) can be inserted therein, stiffening the single diaphragm (10) or double diaphragm (12).

8. Single diaphragm (10) or double diaphragm (12) according to one of the claims 1 to 7, **characterized in that** it is provided with at least one opening (22, 24) for a needle tip or a guide hook.

9. Single diaphragm (10) or double diaphragm (12) according to one of the claims 1 to 8, **characterized in that** it is made of colored PMMA (polymethyl methacrylate) or polycarbonate.

10. Single diaphragm or double diaphragm according to claim 1 or 3, **characterized in that** it is made of a foldable colored material, in particular silicone or soft acrylic.

11. Kit comprising single diaphragms, double diaphragms or one or more single and/or double diaphragms (10, 12) according to one of the claims 1 to 10, **characterized in that** the single and/or double diaphragms (10, 12) can be implanted one above the other with angular offset in the capsule sac, thus overlapping each other such that a central, approximately circular artificial pupil aperture (16) is left open.

12. Kit according to claim 11 for an artificial pupil aperture (16) of 3 mm diameter.

13. Kit according to claim 11 for an artificial pupil aperture (16) of 4 mm diameter.

14. Kit according to one of the claims 11 to 13, **characterized in that** the single and/or double diaphragms (10, 12) can be joined to each other in the intracapsular region, in particular clamped, clipped or snapped together with each other.

15. Fixing ring comprising a flat oval annular body (32) with a central circular aperture (36), on the broad sides of said body (32) two haptic stirrups (34) stand out perpendicular to the plane of the annulus and are bent over outward parallel thereto and can be latched into the overlap region(s) (18) of one or more of the diaphragms (10, 12) according to one of the claims 1 to 14.

## Revendications

1. Diaphragme simple (10) pour créer une ouverture de pupille artificielle à implanter sensiblement sans tension dans la capsule du cristallin d'un oeil en plus d'une lentille artificielle, avec une zone de recouvrement (18) disposée unilatéralement à côté d'un espace libre central (16) et avec un élément de contact (20) en forme d'étrier situé de l'autre côté de celle-ci.

2. Diaphragme simple (10) selon la revendication 1, **caractérisé en ce que** l'élément de contact (20) est configurée en forme de C ou en forme de J.

3. Diaphragme double (12) pour créer une ouverture de pupille artificielle à implanter sensiblement sans tension dans la capsule du cristallin d'un oeil en plus d'une lentille artificielle, avec deux zones de recouvrement (18) opposées l'une à l'autre disposées des deux côtés d'un espace libre central (16) et avec un étrier souple (26) situé latéralement à côté et reliant les zones de recouvrement (18).

4. Diaphragme double (12) selon la revendication 3, **caractérisé en ce que** l'étrier (26) est cintré en forme de U vers l'intérieur.

5. Diaphragme simple ou double (10, 12) selon l'une quelconque des revendications 1 à 4, **caractérisé en ce qu'**il est plat.

6. Diaphragme simple ou double (10, 12) selon l'une quelconque des revendications 1 à 4, **caractérisé en ce qu'**il comprend au moins une zone de recouvrement plate (18) et un élément de contact qui s'en écarte en forme de coude.

7. Diaphragme simple ou double (10, 12) selon l'une quelconque des revendications 1 à 6, **caractérisé en ce qu'**un élément adhésif (28) assurant un raidissement du diaphragme simple ou double (10, 12) peut y être placé.

8. Diaphragme simple ou double (10, 12) selon l'une quelconque des revendications 1 à 7, **caractérisé en ce qu'**il est muni d'au moins une ouverture (22, 24) pour une pointe d'aiguille ou pour un crochet de guidage.

9. Diaphragme simple ou double (10, 12) selon l'une quelconque des revendications 1 à 8, **caractérisé en ce qu'**il se compose de PMMA (polyméthacrylate de méthyle) ou de polycarbonate teinté.

10. Diaphragme simple ou double (10, 12) selon la revendication 1 ou 3, **caractérisé en ce qu'**il se compose d'un matériau repliable et teinté, en particulier du silicone ou de l'acrylique tendre.

11. Lot de diaphragmes simples, de diaphragmes doubles ou d'un ou de plusieurs diaphragme(s) simple(s) et/ou doubles (10, 12) selon l'une quelconque des revendications 1 à 10, **caractérisé en ce que** les diaphragmes simples et/ou doubles (10, 12) peuvent être implantés dans la capsule avec un décalage angulaire et en superposition, alors qu'ils se recouvrent mutuellement de telle façon qu'une ouverture de pupille artificielle approximativement circulaire centrale (16) reste libre.

12. Lot de diaphragmes selon la revendication 11 pour une ouverture de pupille artificielle de 3 mm de diamètre.

13. Lot de diaphragmes selon la revendication 11 pour une ouverture de pupille artificielle de 4 mm de diamètre.

14. Lot de diaphragmes selon l'une quelconque des revendications 11 à 13, **caractérisé en ce que** le diaphragme simple et/ou les diaphragmes doubles (10, 12) peut(peuvent) faire l'objet d'une liaison intracapsulaire réciproque et peut(peuvent) en particulier être réciproquement tendu(s), clipsé(s) ou relié(s) par crantage.

15. Anneau de fixation avec un corps de bague plat et ovale (32) ayant une ouverture centrale circulaire (36) et sur les côtés larges duquel deux élément de contact en forme d'étrier (34) sont recourbés vers l'extérieur perpendiculairement à partir du plan de l'anneau en hauteur et parallèlement à celui-ci, lesquels éléments de contact peuvent être encliquetés sur la(les) zone(s) de recouvrement (18) d'un ou de plusieurs diaphragme(s) (10, 12) selon l'une quelconque des revendications 1 à 14.
